Europäisches Patentamt

European Patent Office

Office européen des brevets

(19)

(11) Publication number : **0 485 323 A1**

(12) **EUROPEAN PATENT APPLICATION**

(21) Application number : **91630099.9**

(22) Date of filing : **07.11.91**

(51) Int. Cl.⁵ : **A61N 5/02, A61B 5/06, A61F 7/12**

(30) Priority : **09.11.90 IL 96289**

(43) Date of publication of application :
**13.05.92 Bulletin 92/20**

(84) Designated Contracting States :
**AT BE CH DE DK ES FR GB IT LI NL SE**

(71) Applicant : **BIODAN MEDICAL SYSTEMS LTD**
**Kiryat Weizmann, P.O. Box 2012**
**Rehovot 76120 (IL)**

(72) Inventor : **Taicher, Zvi**
**4 Ben Yehuda St.**
**Rehovot (IL)**
Inventor : **Leib, Zvi**
**42 Bernstein St.**
**Ramat Chen, Ramat Gan (IL)**

(74) Representative : **Waxweiler, Jean et al**
**OFFICE DENNEMEYER & ASSOCIATES Sàrl,**
**P.O. Box 1502**
**L-1015 Luxembourg (LU)**

(54) **Hyperthermia apparatus.**

(57) Hyperthermia apparatus for heating predetermined tissue in the body of a subject includes a probe device (3) and heat applicator (2) each positionable in proximity to the tissue to be heated, the location of one of which is known and that of the other is unknown. The apparatus further includes distance measuring means (45) for measuring the distance between the heat applicator device (2) and probe device (3) when positioned with respect to the tissue to be heated, and thereby to indicate the location of the device of initially unknown location. The probe device (3) and heat applicator device (2) also include temperature sensors (25a-c, 32a-c), thereby enabling mapping the temperature field in the area adjacent the tissue to be heated.

FIG 4

EP 0 485 323 A1

Jouve, 18, rue Saint-Denis, 75001 PARIS

The present invention relates to hyperthermia apparatus, and particularly to apparatus for administering a hyperthermia treatment to predetermined tissue in the body of a subject. The invention is particularly useful for administering a hyperthermia treatment to the prostate of a subject, and is therefore described below with respect to this application.

Hyperthermia is a recognized technique for various therapeutic modalities in which the tissue temperature is elevated by the application of heat. In one known type of apparatus, a heat applicator is inserted via the patient's rectum to heat the prostate, and a probe is inserted via the subject's urethra to precisely locate the heat applicator and to precisely measure the temperature at several points in or adjacent the treated site, in order to measure the effectiveness of the hyperthermia treatment and also to protect the patient against damage to other tissue. One known heat applicator which includes a heater in the form of a microwave antenna is described in Application No.87630080.7; and one known type of probe for use with such heat applicators is described in Application No.87630081.5.

The temperature field produced by the application of local microwave heating is a complicated function of tissue parameters, such as geometry, electromagnetic properties, heat transfer constants, blood flow, etc. At the location being heated, these parameters may vary widely with each subject, which makes it difficult to map the temperature field in the area adjacent to the tissue to be heated in order to maximize the effectiveness of the heat treatment. Moreover, some of these parameters are dependent on time and temperature, which even more complicates the mapping of the temperature field.

In one known technique of temperature mapping, a needle is inserted under ultrasonic or X-ray guidance at a preselected point and then replaced by a temperature sensor before the application of the heat, since no metal is allowed to be in the vicinity of the heat applicator. A serious disadvantage of this technique, however, is that during the application of the heat the temperature sensor may move and therefore would not indicate the true condition of the temperature field.

An object of the present invention is to provide hyperthermia apparatus which better enables the position of the heat applicator to be determined not only before the hyperthermia treatment, but also during the treatment. Another object of the invention is to provide hyperthermic apparatus which better enables mapping the temperature field in the area adjacent the tissue to be heated.

According to the present invention, there is provided hyperthermia apparatus for heating predetermined tissue in the body of a subject, comprising: a probe device positionable in proximity to the tissue to be heated; a heat applicator device positionable adjacent to, the tissue to be heated; the location of one of the devices being known and that of the other being unknown; and distance measuring means for measuring the distance between the heat applicator device and probe device when positioned with respect to the tissue to be heated, and thereby to indicate the location of the device of initially unknown location.

According to a further feature in the described preferred embodiment, the probe device also includes a temperature sensor, thereby enabling mapping the temperature field in the area adjacent the tissue to be heated.

It has been found that the foregoing apparatus enables mapping the temperature field in the area adjacent to the area to be heated during the heating operation, thereby making the apparatus particularly useful for administering a hyperthermia treatment to predetermined tissue (e.g., the prostate) in the body of a person.

Further features and advantages of the invention will be apparent from the description below.

The invention is herein desribed, by way of example only, with reference to the accompanying drawings, wherein:

Fig. 1 illustrates one form of apparatus in accordance with the present invention for use in administering hyperthermia to the prostate of a subject;

Fig. 2 is a side elevational view illustrating the heat applicator in the apparatus of Fig. 1;

Fig. 3 diagrammatically illustrates a part of the probe in the apparatus of Fig. 1;

Fig. 4 is a block diagram illustrating the main components in the apparatus of Fig. 1; and

Fig. 5 is a block diagram illustrating a portion of the circuit in the apparatus of Fig. 4.

As indicated earlier, the apparatus illustrated in the drawings is particularly useful for administering a hyperthermia treatment to the prostate P of a subject. For this purpose, it includes a heat applicator, generally designated 2, insertable into the rectum R of the subject; and a probe 3 movable through a catheter 4 insertable into the urethra for precisely locating the position of the heat applicator 2 in order to maximize the effectiveness of the hyperthermia treatment. The heat applicator 2, probe 3 and catheter 4 are generally of the constructions described in the above-cited patent applications. As described therein, the end of the catheter 4 is inserted into the bladder B of the subject, and includes a balloon 5 which is inflated via an inlet fitting 5a in order to fix the location of the end of the catheter with respect to the prostate P.

The heat applicator 2 also includes a balloon 6 laterally of its tip, which balloon is inflated in order to firmly engage the tissue in the rectum R. Balloon 6 thus presses the respective end of the heat applicator 2 towards the prostate to be heated and also firmly anchors the heat applicator in this position during the hyperthermia treatment.

Catheter 4 is a long slender tube insertable through the urethra U and includes three passageways: a first passageway for inserting the probe 3; a second passageway for inflating the balloon 5 in the neck of the bladder B in order to fix the position of the end of the catheter; and a third passageway for draining the bladder B during the administration of the hyperthermia treatment, all as described more particularly in the above-cited patent applications.

Thus, during the administration of the hyperthermia treatment to the prostate P, the positions of the catheter 4, and thereby of the probe 3, are known parameters. A further known parameter is the temperature at the known position of the probe 3, since the probe includes a temperature sensor, as described in the above-cited patent applications. As distinguished from the apparatus described in those patent applications, the apparatus described herein further includes means for precisely measuring the distance between the probe 3 and the heat applicator 2 in order to position the heat applicator for maximum effectiveness of the hyperthermia treatment of the prostate.

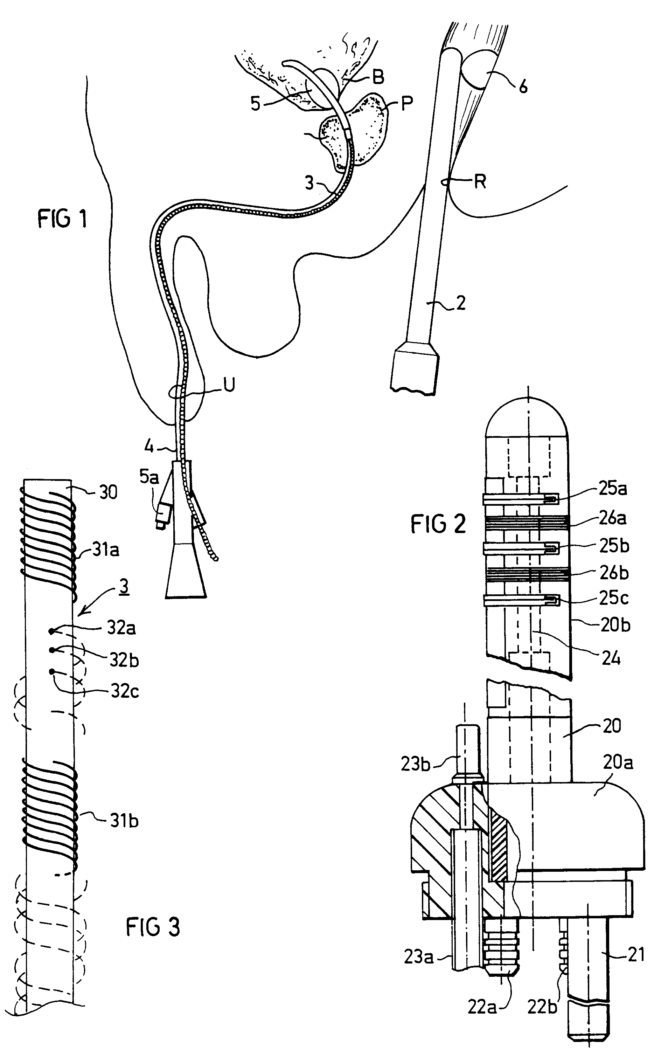

The heat applicator, as more particularly illustrated in Fig. 2, includes a housing 20 formed with an enlarged base 20a and a long narrow stem 20b insertable into the rectum R of the subject. The base 20a includes an electrical fitting 21 for making the electrical connections to the heat applicator, a pair of water cooling fittings 22a, 22b for circulating cooling water through the heat applicator, and an inflation fitting 23a for inflating through nipple 23b the balloon 6 applied, via a flexible sleeve (not shown), to the end of the heat applicator in order to firmly anchor it in position in the rectum.

The applicator 2 illustrated in Fig. 2 further includes a dipole microwave transmitter antenna 24 for heating the prostate P during the hypethermia treatment, and a plurality of temperature sensors (thermocouples) 25a-25c for measuring the temperature at the surface of the heat applicator.

The construction and operation of the heat applicator 2, insofar as described above, are more particularly set forth in the above-cited Application No.87630080.7, which description is hereby incorporated by reference.

According to the present invention, the heat applicator 2 further includes low frequency transmitters or radiators, indicated by coils 26a, 26b, which transmit low frequency electromagnetic waves in order to permit the heat applicator 2 to be precisely positioned, and its position to be precisely determined relative to the probe so as to enable mapping the temperature field of the area of the prostate adjacent to the temperature sensors of the probe.

The probe 3, as illustrated in Fig. 3, is made of a cylindrical body 30 of dielectric material. It includes low frequency receiving coils 31a, 31b tuned to receive the electromagnetic waves transmitted by the low frequency transmitter coils 26a, 26b of the heat applicator 2, and a plurality of temperature sensors (thermocouples) 32a-32c for sensing the temperature at the respective locations of the probe. In all other respects, the probe 3 is constructed and operates in the same manner as described in our prior Application No.87630081.5, which is hereby incorporated by reference.

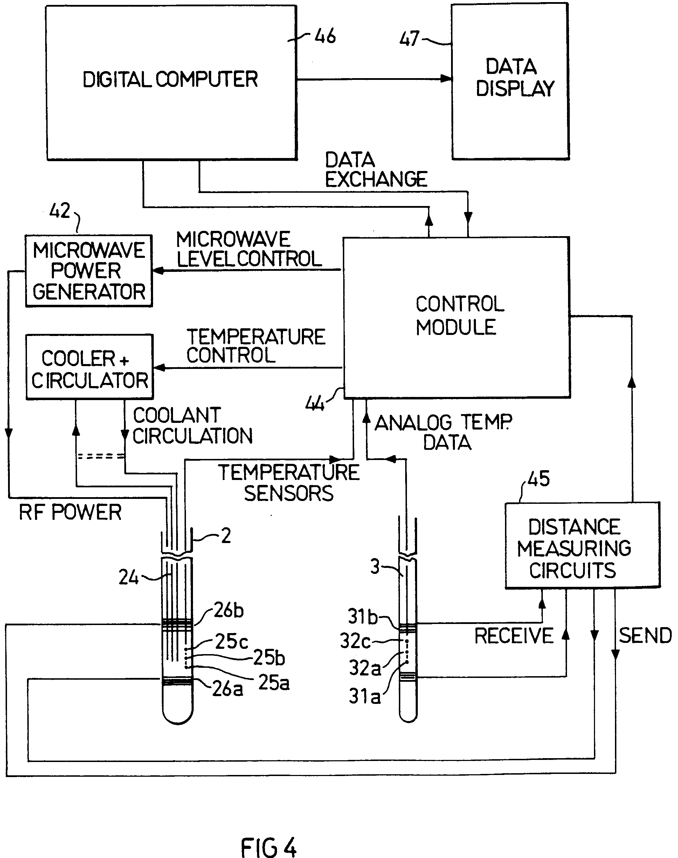

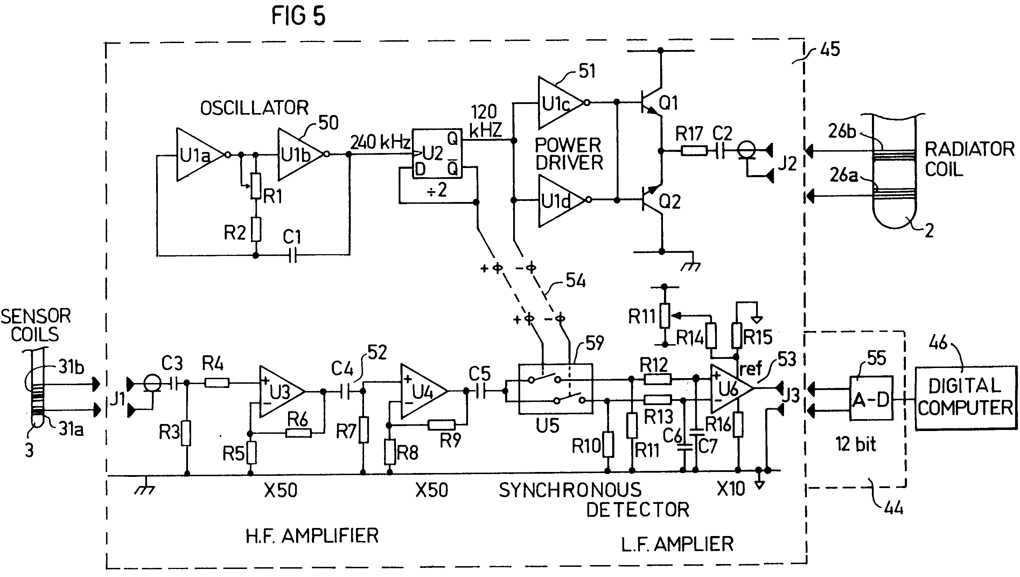

Fig. 4 illustrates the overall hyperthermia apparatus, and Fig. 5 more particularly illustrates the system for measuring the distance between the low frequency transmitter coils 26a, 26b of the heat applicator 2, and the low frequency receiver coils 31a, 31b of the probe 3.

Thus, as shown in Fig. 4, the microwave transmitter antenna 24 is supplied from a microwave power generator 42. The heat applicator 2 is cooled by a cooling fluid (e.g., water) circulated by a coolant source 43. The temperature sensed by the thermocouples 25a-25c of the heat applicator 2 is fed to a control module 44. Control module 44 also senses and controls the power of the microwave frequency power generator 42, and also senses and controls the temperature of the coolant circulated by circulator 43.

The temperature sensed by thermocouples 32a-32c of the probe 3 is also fed to control module 44.

The distance between the probe 3 and the heat applicator 2 is measured by a distance measuring circuit 45. For this purpose, circuit 45 measures the magnitude of the low frequency electromagnetic wave transmitted by coils 26a, 26b in the heat applicator 2 received by coils 31a, 31b in the probe 3. This measured signal is fed to the control module 44 connected to a digital computer 46. A look-up table of previously calculated data is stored in computer 46, and the measurement produced by circuit 45 is compared to the values in this table to permit an accurate determination to be made of the distance between the transmitters coils 26a, 26b in the heat applicator 2 and the receiver coils 31a, 31b in the probe 3. Digital computer 46 is also connected to a data display 47 for displaying this information.

The distance measuring circuit 45 in Fig. 4 is more particularly illustrated in Fig. 5. Thus, the low frequency transmitter coils 26a, 26b of the heat applicator 2 are supplied from an oscillator 50 and a power driver 51. The electromagnetic energy received by the receiver coils 31a, 31b in the probe 3 is amplified in a high frequency amplifer 52 and is synchronously detected by detector 59, which received a reference signal via a tap 54. The resulting signal is amplified in a low frequency amplifier 53, and the resulting magnitude is converted to digital form in an A/D converter 55. Converter 55 is a part of the control module 44 feeding the computer 46 for processing, as described above.

The manner of using the illustrated hyperthermia apparatus will be apparent from the above description.

Thus, the catheter 4 is first inserted into the subject's urethra U through the prostate P and into the bladder B, and then the balloon 5 is inflated by introducing water (or air) via the inlet fitting 5a. The catheter is then slightly withdrawn until the inflated balloon 5 limits against the neck of the bladder B as shown in Fig. 1.

Probe 3 is then inserted into the catheter a predetermined (known) distance such that its low frequency receiver antenna 31 is located substantially centrally of the prostate P. Normally, this is to be the direction of transmitting the microwave electromagnetic field to be produced by the heat applicator 2.

The heat applicator 2 is then inserted into the patient's rectum R while very low power is transmitted via its low frequency transmitter coils 26a, 26b, until a maximum of an appropriate combination of readings is produced by the low frequency receiver coils 31a, 31b of the probe 3. This indicates that the heat applicator is precisely located such that the direction of transmitting the microwave electromagnetic field produced by it is substantially at the center of the prostate.

The microwave transmitter antenna 24 may then be energized to produce the microwave electromagnetic field for administering the hyperthermia treatement to the prostate P. The temperature sensors 25a-25c carried by the heat applicator 2, and the temperature sensors 32a-32c carried by the probe 3, measure the temperature at these known points with respect to the prostate P, the heat applicator 2, and the probe 3. This enables the temperature field in the area within and adjacent the proste P to be mapped, thereby also enabling the effectiveness of the hyperthermia treatment to be maximized according to the particular case.

The low frequency electromagnetic field used for measuring the distance between the heat applicator and the probe is preferably between 50 and 150 KHz, while the frequency of the microwave transmitter antenna is preferably about 915 MHz. It has been found that when using a low frequency transmission below 50 KHz, the sensitivity drops, and above 150 KHz, the distance-measurement may be disturbed by the environment influences, and particularly by the attenuation of the signal by the subject's body, therefore introducing errors in the distance measurement. Best results have been found when the low frequency transmission is about 120 KHz.

As one example, temperature sensor 25b (Fig. 2) may be located at the center of the transmitter antenna, and the two low frequency coils 26a, 26b may be located at equal distances on opposite sides of the antenna center. Similarly, temperature sensor 25b may also be located at the antenna center, and temperature sensors 25a, 25c may be located at equal distances on opposite sides of the antenna center. The low frequency receiving coils 31a, 31b may be located on the probe 3 so that the distance between their centers is equal to the distance between the centers of the transmitter coils 26a, 26b. The center between the two receiving coils 31a, 31b is to be located substantially in the central area of the prostate and is the target to be heated. Temperature sensor 32b may be located at the center between the two receiving coils 31a and 31b, and the two temperature sensors 32a and 32c may also be located at equal distances on opposite sides of the temperature sensor 32b.

The above positioning of the heat applicator maximizes the effectiveness of the hyperthermia treatment. The distance between the centers of the applicator 2 and the probe 3 are correlated to the signal amplitude outputted from the A-D converter 55 with the look-up table in the computer 46 of the signal amplitudes related to distance previously precalibrated, to enable a determination to be made of the distance between the centers of the low frequency transmitter coils of the applicator and the low frequency receiver coils of the probe. This measurement, together with the measurements of the temperature at a plurality of points on the probe and on the applicator, permit mapping the temperature field in the area within and adjacent the prostate to be heated.

It will thus be seen that the apparatus described above permits the applicator to be positioned for maximizing the effectiveness of hyperthermia treatment and also permit mapping of the temperature field in and around the prostate, thereby not only enabling the hyperthermia treatment to be maximized, but also to be monitored so as not to unduly overheat and damage tissues not to be heated above preset limits.

## Claims

1. Hyperthermia apparatus for heating predetermined tissue in the body of a subject, comprising:
   a probe device positionable in proximity to the tissue to be heated;
   a heat applicator device positionable in proximity to, the tissue to be heated;
   the location of one of said devices being known and that of the other being unknown;
   and distance measuring means for measuring the distance between said heat applicator device and probe device when positioned with respect to the tissue to be heated, and thereby to indicate the location of the device of initially unknown location.

2. The apparatus according to Claim 1, wherein said probe device and heat applicator device also include temperature sensors, thereby enabling mapping the temperature field in the area adjacent the tissue to be heated.

3. The apparatus according to Claim 1, wherein said distance measuring means includes at least one low frequency electromagnetic wave transmitter coil carried by the heat applicator and at least one low frequency electromagnetic wave receiver coil carried by the probe.

4. The apparatus according to Claim 3, wherein said heat applicator device includes a microwave transmitter antenna.

5. The apparatus according to Claim 3, wherein said low frequency transmitter coil is energized at 50-150 KHz.

6. The apparatus according to Claim 3, wherein said distance measuring means includes means for measuring the magnitude of the low frequency electromagnetic wave received by the probe as compared to a predetermined stored value.

7. The apparatus according to Claim 1, wherein said apparatus further includes a catheter insertable into a body cavity, said probe device being insertable into and through said catheter.

8. The apparatus according to Claim 7, wherein said catheter is a long slender tube insertable via the subject's urethra into the subject's bladder and includes an inflatable balloon for precisely locating the probe, and thereby the heat applicator device, with respect to the subject's prostate.

9. The apparatus according to Claim 8, wherein said catheter also includes a passageway for draining the subject's bladder, and a passageway for inflating the balloon.

FIG 1

FIG 2

FIG 3

FIG 4

FIG 5

EP 0 485 323 A1

European Patent
Office

**EUROPEAN SEARCH REPORT**

Application Number

EP    91 63 0099

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y,D | EP-A-0 246 176 (BIODAN MEDICAL SYSTEMS LTD)<br>* claims 1,2; figure 8 *<br>--- | 1-9 | A61N5/02<br>A61B5/06<br>A61F7/12 |
| Y | EP-A-0 359 697 (PHARMACIA DELTEC,INC.)<br>* column 3, line 15 - line 34 *<br>* line 50 - line 53 *<br>--- | 1-9 | |
| A,D | EP-A-0 248 758 (BIODAN MEDICAL SYSTEMS LTD)<br>* page 3, line 31 - page 4, line 1 *<br>--- | 1,4 | |
| A | US-A-4 173 228 (VAN STEENWIJK ET AL.)<br><br>----- | | |
| | | | **TECHNICAL FIELDS SEARCHED (Int. Cl.5 )**<br><br>A61N<br>A61B<br>A61F |

The present search report has been drawn up for all claims

| Place of search | Date of completion of the search | Examiner |
|---|---|---|
| THE HAGUE | 14 FEBRUARY 1992 | GLAS J. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)